# EUROPEAN PATENT APPLICATION

(11) **EP 4 591 861 A1**
(43) Date of publication of application: **30.07.2025**
(21) Application number: 23867538.3
(22) Date of filing: 20.09.2023
(51) Int. Cl.: A61K 31/205, A61P 25/24

(54) **USE OF 4-TMAP FOR TREATING OR RELIEVING DEPRESSION**

(30) Priority: 23.09.2022 CN 202211165418
(71) Applicant: IBIOME Biotechnology Co., Ltd., Hefei, Anhui 230601 (CN)
(72) Inventor: ZHU, Shu, Hefei, Anhui 230601 (CN); HU, Ji, Hefei, Anhui 230601 (CN); HUANG, Chuan, Hefei, Anhui 230601 (CN); TAO, Wanyin, Hefei, Anhui 230601 (CN)
(74) Representative: Ipside
(86) International application number: PCT/CN2023/119895
(87) International publication number: WO 2024/061251

(57) **Abstract**

This invention discloses a new use of 4-(trimethylammonio) pentanoate (4-TMAP), specifically relating to the application of 4-(trimethylammonio) pentanoate in the preparation of drugs for treating depression. The invention also provides pharmaceutical compositions containing 4-(trimethylammonio) pentanoate and their applications in the preparation of antidepressant medications. This invention not only enriches the existing antidepressant drug technology but also develops new indications for 4-(trimethylammonio) pentanoate, holding potential economic and social value.

## Description

### Technical Field

The invention relates to the field of pharmaceutical chemistry, specifically to the use of 4-TMAP in the preparation of drugs for treating or alleviating depression.

### Background of the Invention

Depression is a common mental disorder characterized by low mood, insomnia, loss of appetite, and difficulty concentrating. Severe cases increase the risk of suicide, significantly affecting patients' well-being. According to the World Health Organization, over 320 million people worldwide suffer from depression. Studies predict that by 2030, depression will become a major disease burden in middle- and high-income countries. Therefore, understanding the pathogenesis of depression and finding effective treatments is an urgent need.

Currently, commonly used antidepressants include monoamine oxidase inhibitors (MAOls), norepinephrine and dopamine reuptake inhibitors (NDRIs), selective serotonin reuptake inhibitors (SSRIs), serotonin and norepinephrine reuptake inhibitors (SNRIs), serotonin antagonists and reuptake inhibitors (SARIs), tricyclic antidepressants (TCAs), and tetracyclic antidepressants. These drugs exert antidepressant effects by enhancing the function of serotonin, dopamine, and norepinephrine. Despite their importance in treating depression, they often cause severe side effects, including headaches, dizziness, gastrointestinal discomfort, sexual dysfunction, and blood pressure or weight abnormalities. Additionally, these drugs have long onset times, poor population drug sensitivity, high costs, and strong drug resistance. These limitations may arise from weak target specificity and unclear mechanisms of action.

With the increasing number of depression patients, current antidepressant drugs struggle to meet clinical needs. Therefore, finding novel antidepressants with significant efficacy, fewer side effects, and clear mechanisms is crucial.

4-(trimethylammonio) pentanoate (4-TMAP) is a bacterial metabolite discovered in mice and a structural analog of carnitine. Its structure is shown in formula (I):

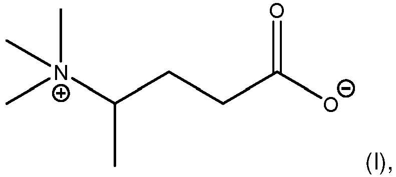

4-TMAP is found in the intestines and brains of specific pathogen-free mice and acts as an analog of mildronate. Mildronate is an effective inhibitor of human GBB hydroxylase (BBOX1) and carnitine acetyltransferase (CrAT), enzymes involved in carnitine synthesis and fatty acid transport into mitochondria. 4-TMAP binds to the active site of human BBOX1 with nearly the same conformation as mildronate and has a low dissociation constant. Exploring the additional biological activities of known compounds to discover new medical values is an essential aspect of drug discovery.

### Summary of the Invention

The primary objective of this invention is to provide a new application of 4-TMAP in the preparation of drugs for treating or alleviating depression. This invention expands the indications of this compound and offers a clinical solution for depression to meet market demand.

To achieve this objective, the technical solution adopted by the invention includes:
The use of 4-(trimethylammonio) pentanoate (4-TMAP) or its pharmaceutically acceptable salts in the preparation of drugs for treating or alleviating depression.

Further, this invention provides a use of 4-(trimethylammonio) pentanoate (4-TMAP) or its pharmaceutically acceptable salts in the preparation of drugs for treating or alleviating depression in mammals, preferably the mammals include primates and rodents, and more preferably the mammals include humans, monkeys, or mice.

Further, this invention provide a pharmaceutical composition containing 4-(trimethylammonio) pentanoate (4-TMAP) or its pharmaceutically acceptable salts thereof, the pharmaceutical composition comprising a therapeutically effective amount of 4-(trimethylammonio) pentanoate (4-TMAP) or its pharmaceutically acceptable salts thereof; the preferred therapeutically effective dose is ≥0.1 mg/kg per dose for mammals.

Further, the pharmaceutical composition of this invention comprises pharmaceutically acceptable excipients, carriers, adjuvants, or media.

Examples of excipients, carriers, adjuvants, or media include, but are not limited to, anti-adherents, binders, coatings, compression aids, disintegrants, dyes, lubricants, emulsifiers, fillers (diluents), film-forming agents, flavoring agents, flow aids, stabilizers, and vitamins. Specific examples include BHT, calcium carbonate, calcium phosphate (monohydrate), calcium stearate, croscarmellose sodium, cross-linked polyvinylpyrrolidone, citric acid, cross-linked povidone, cysteine, ethylcellulose, gelatin, hydroxypropylcellulose, hydroxypropyl methylcellulose, lactose, magnesium stearate, maltitol, mannitol, methionine, methylcellulose, methylparaben, microcrystalline cellulose, polyethylene glycol, povidone, pregelatinized starch, propylparaben, retinyl palmitate, shellac, silica, sodium carboxymethylcellulose, sodium citrate, sodium starch glycolate, sorbitol, starch (corn), stearic acid, stearic acid, sucrose, talc, titanium dioxide, vitamin A, vitamin E, vitamin C, and xylitol.

The pharmaceutical composition may be prepared in the form of injectable or oral formulations. Injectable formulations may be classified based on their state as liquid injections, powder injections, or tablet injections. They may also be classified by injection site, including intradermal, subcutaneous, intramuscular, intravenous, spinal, or intra-articular injections. Preferably, the solvent for injectable formulations includes sterile water for injection or normal saline solution.

Oral formulations include tablets containing the active ingredient mixed with non-toxic pharmaceutically acceptable excipients, such as inert diluents or fillers (e.g., sucrose, sorbitol, sugar, mannitol, microcrystalline cellulose, starch, calcium carbonate, sodium chloride, lactose, calcium phosphate, or sodium phosphate). Granulating and disintegrating agents (e.g., cellulose derivatives including microcrystalline cellulose, starch including potato starch, cross-linked carboxymethyl cellulose sodium, alginates or alginic acid); binders (e.g., sucrose, glucose, sorbitol, gum arabic, alginic acid, sodium alginate, gelatin, starch, pregelatinized starch, microcrystalline cellulose, magnesium aluminum silicate, sodium carboxymethyl cellulose, methylcellulose, hydroxypropyl methylcellulose, ethylcellulose, polyvinyl pyrrolidone or polyethylene glycol); and lubricants, glidants and anti-adherents (e.g., magnesium stearate, zinc stearate, stearic acid, silicon dioxide, hydrogenated vegetable oil or talc). Preparations for oral use may also be in the form of chewable tablets, or in the form of hard gelatin capsules in which the active ingredient is mixed with an inert solid diluent (e.g., potato starch, lactose, microcrystalline cellulose, calcium carbonate, calcium phosphate or kaolin), or in the form of soft gelatin capsules in which the active ingredient is mixed with water or an oil medium (e.g., peanut oil, liquid paraffin or olive oil). Powders, granules and pills can be prepared using the ingredients mentioned above under tablets or capsules in a conventional manner using, for example, a mixer, fluidized bed equipment or spray drying equipment.

Other pharmaceutically acceptable excipients for oral formulations include, but are not limited to, colorants, flavoring agents, plasticizers, humectants, and buffers. Formulations for oral use may also be in the form of chewable tablets, or in the form of hard gelatin capsules, wherein the active ingredient is mixed with an inert solid diluent (e.g., potato starch, lactose, microcrystalline cellulose, calcium carbonate, calcium phosphate, or kaolin), or in the form of soft gelatin capsules, wherein the active ingredient is mixed with water or an oil medium (e.g., peanut oil, liquid paraffin, or olive oil). Powders, granules, and pills may be prepared in a conventional manner using, for example, a mixer, a fluidized bed apparatus, or a spray drying apparatus using the ingredients mentioned above under tablets or capsules.

In some embodiments, administration comprises administering a composition described herein intramuscularly, intravenously (e.g., in the form of a sterile solution and in a solvent system suitable for intravenous use), intradermally, intraarterially, intraperitoneally, intralesionally, intracranially, intraarticularly, intraprostatically, intrapleurally, intratracheally, intranasally, intravitreally, intravaginally, intrarectally, topically, intratumorally, transperitoneally, subcutaneously, subconjunctivally, intracapsularly, transmucosally, intrapericardially, intraumbilically, intraocularly, orally (e.g., as a tablet, capsule, caplet, caplet-shaped tablet, or syrup), topically (e.g., in the form of a cream, gel, lotion, or ointment), topically, by inhalation, by injection, or by infusion (e.g., continuous infusion in the form of a cream or lipid composition, local perfusion, catheterization, lavage directly bathing target cells).

Compared with the existing technology, this invention has the following advantages:
The small-molecule drug that can treat or alleviate depression disclosed in this invention, can significantly improve the depressive symptoms in different depression models in mice, which is manifested in reducing the immobility time of mice in forced swimming and tail suspension experiments, improving the sugar water preference of mice. At the same time, compared with the positive control brexpiprazole, it has the characteristics of lower dosages, stronger efficacy and offering prolonged effects.

This invention expands the new solutions for treating or relieving depression in the prior art, and has potential commercial development value. The present invention discovers a new use of 4-(trimethylammonio) pentanoate (4-TMAP) through preliminary screening, providing new solutions for developing better antidepressant drugs.

### Brief Description of the Drawings

FIG. 1 shows the changes in the content of 4-TMAP in brain tissue at different time points after intraperitoneal injection of 4-TMAP in Example 1.
FIG. 2 shows the effect of 4-TMAP administration on forced swimming of mice in the CRS-induced depression model in Example 2, and the numbers marked between the groups in the figure represent the *P* values between the two groups.
FIG. 3 shows the effect of 4-TMAP administration on tail suspension of mice in the CRS-induced depression model in Example 2, and the numbers marked between the groups in the figure represent the *P* values between the two groups.
FIG. 4 shows the effect of 4-TMAP administration on sucrose preference of mice in the CRS-induced depression model in Example 2, and the numbers marked between the groups in the figure represent the *P* values between the two groups.
FIG. 5 shows the effect of 4-TMAP administration on forced swimming of mice in the Cort-induced depression model in Example 3, and the numbers marked between the groups in the figure represent the *P* values between the two groups.
FIG. 6 shows the effect of 4-TMAP administration on tail suspension of mice in the Cort-induced depression model in Example 3, and the numbers marked between the groups in the figure represent the *P* values between the two groups.
FIG. 7 shows the effect of 4-TMAP administration on sucrose preference of mice in the Cort-induced depression model in Example 3, and the numbers marked between the groups in the figure represent the *P* values between the two groups.
FIG. 8 shows the effect of 4-TMAP administration on forced swimming of mice in the AAD-induced depression model in Example 4, and the numbers marked between the groups in the figure represent the *P* values between the two groups.
FIG. 9 shows the effect of 4-TMAP administration on tail suspension of mice in the AAD-induced depression model in Example 4, and the numbers marked between the groups in the figure represent the *P* values between the two groups.
FIG. 10 shows the effect of 4-TMAP administration on sucrose preference of mice in the AAD-induced depression model in Example 4, and the numbers marked between the groups in the figure represent the *P* values between the two groups.
FIG. 11 shows the minimum dose of 4-TMAP for relieving depression-like behavior in mice detected by the forced swimming test in Example 5, and the numbers marked between the groups in the figure represent the *P* values between the two groups.
FIG. 12 shows the minimum dose of 4-TMAP for relieving depression-like behavior in mice detected by the tail suspension test in Example 5, and the numbers marked between the groups in the figure represent the *P* values between the two groups.
FIG. 13 shows the minimum dose of 4-TMAP for relieving depression-like behavior in mice detected by the sucrose preference experiment in Example 5, and the numbers marked between the groups in the figure represent the *P* values between the two groups.
FIG. 14 is the forced swimming test in Example 6 to monitor the duration of depression-like behavior in mice treated with 4-TMAP once, and the numbers marked between the groups in the figure represent the *P* values between the two groups.
FIG. 15 is the tail suspension test in Example 6 to monitor the duration of depression-like behavior in mice treated with 4-TMAP once, and the numbers marked between the groups in the figure represent the *P* values between the two groups.
FIG. 16 is the forced swimming test in Example 7 to compare the antidepressant effects of 4-TMAP and Brexpiprazole, and the numbers marked between the groups in the figure represent the *P* values between the two groups.
FIG. 17 is the tail suspension test in Example 7 to compare the antidepressant effects of 4-TMAP and Brexpiprazole, and the numbers marked between the groups in the figure represent the *P* values between the two groups.
FIG. 18 is the sucrose preference test in Example 7 to compare the antidepressant effects of 4-TMAP and Brexpiprazole, and the numbers marked between the groups in the figure represent the *P* values between the two groups.

### Detailed Description of Embodiments

To make the technical means, creative features, objectives and effects achieved by the present invention easy to understand, the present invention will be further elaborated below in conjunction with specific embodiments.

The present invention discloses a novel antidepressant. Three different depression models are established in mice: a depression model of chronic unpredictable stress (CRS), a depression model induced by corticosterone (Cortitone), and a depression model induced by amino acid food (AAD). The antidepressant effect of small molecule drugs in mice is detected by behavioral detection indicators: forced swimming test (FST), tail suspension test (TST), and sucrose preference test (SPT). At the same time, the antidepressant effect and the duration of the antidepressant effect are compared with those of the third-generation antipsychotic drug Brexpiprazole commonly used in clinical practice.

### Example 1: 4-TMAP can cross the blood-brain barrier through blood circulation and exert antidepressant effects

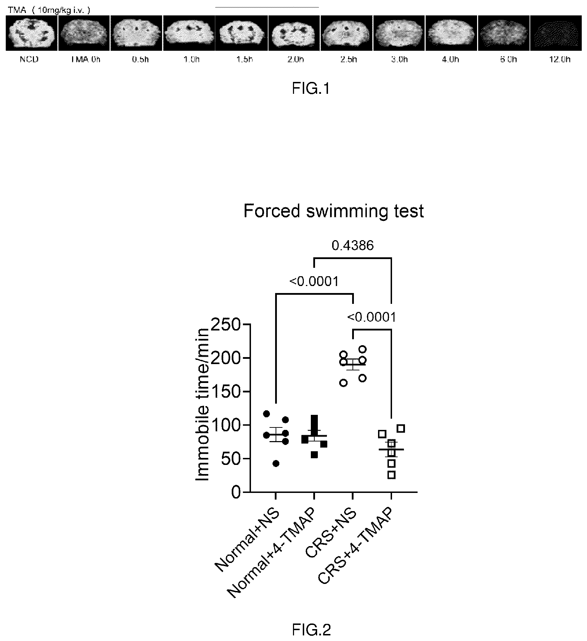

Mice were injected intraperitoneally with 4-TMAP (relative dose 10 mg/kg), and serum and brain tissues of the mice were collected at 10 time points after administration, namely 0h, 0.5h, 1h, 1.5h, 2.0h, 2.5h, 3.0h, 4.0h, 6h, and 12h. Small molecules in the serum were extracted according to the extraction method of polar substances, and the fresh brains were immediately frozen in liquid nitrogen to make frozen sections (10µm). The changes of 4TMAP over time were detected by IMS imaging mass spectrometry, as shown in FIG. 1, indicating that 4-TMAP can directly enter the brain tissue through the blood-brain barrier with systemic blood circulation to exert its effect.

### Example 2: Effect of 4-TMAP in a mouse model of depression induced by chronic unpredictable stress (CRS)

Twenty-four 6-8 week old SPF grade C57/B6j male mice (purchased from GemPharmatech) were randomly divided into four groups: one group was a normal mouse + normal saline group (6 mice), one group was a normal mouse + 4-TMAP group (6 mice), one group was a CRS mouse + normal saline group (6 mice), and one group was a CRS mouse + 4-TMAP group (6 mice). After adapting to the environment, from day 0, the CRS mouse group was subjected to chronic, unpredictable mild stimulation for 21 days to simulate the chronic low-intensity stress received by humans in daily life. Every day, one stimulus was selected from the day and night to treat the mice, and the order was random and continuous without repetition, so that the mice could not predict the appearance of the stimulus (Table 1). After 21 days of continuous modeling, the depression modeling was confirmed to be successful by forced swimming, tail suspension, and sucrose preference tests (significant differences from normal mice).

**Table 1**

| Stressor | Description |
|---|---|
| Fasting food and water | Fasting food or water separately or simultaneously are used separately |
| Forced Swimming | Mice swim in 25°C water for 20-30 min |
| Tilt cage | The mouse cage is tilted 45°, usually after fasting food and water, to increase the difficulty of mice to eat and drink. |
| Wet cage | Pour 250ml of water into the bedding |
| Day and night reversal | 12h day and night reversal |
| Strobe | 120 flashes/min, 6 h |

| Restraint | Mice was placed in a restrainer (20 cm long, 8 cm in diameter) and restrained for 2-4 hours. |
|---|---|
| Intermittent lighting | 2h light and dark alternation |
| Noise | 85 dB, 30 min |

The mice in the 4-TMAP group were given 200 µL 4-TMAP (0.1 mg/kg) by intravenous injection at the ocular margin, and the mice in the normal saline group were given 200 µL normal saline. Two hours after administration, the mice were subjected to forced swimming and tail suspension tests, and the immobility time of the mice in each group in the forced swimming and tail suspension tests was compared. The specific experimental methods are as follows.

The forced swimming test apparatus is a transparent 20×30cm round barrel filled with pure water, with a diameter of 20cm and a water depth of 15cm. The pure water temperature is 23-25°C. A camera is installed in front of the apparatus and is flush with the water surface. After the mouse is placed in the water, a video of its activities in the water is collected for 6 minutes. The last 4 min of the video were analyzed to determine the immobility time of the mouse. Immobility behavior is defined as the behavior of the mouse floating, not struggling, or relying only on occasional swinging to maintain floating. The longer the immobility time, the more severe the depression.

The tail suspension test uses tape to suspend mice by their tails in a position where they cannot escape or grab onto nearby surfaces. A camera is mounted in front of the mice to record the activity of the mice for 6 minutes while they are suspended. The last 4 min of the video is then analyzed to determine the time the mice spend immobile. This test is based on the idea that animals that are subjected to short-term, unavoidable stress will develop an immobile posture, and the more severe the depressive phenotype, the less time they will spend trying to escape.

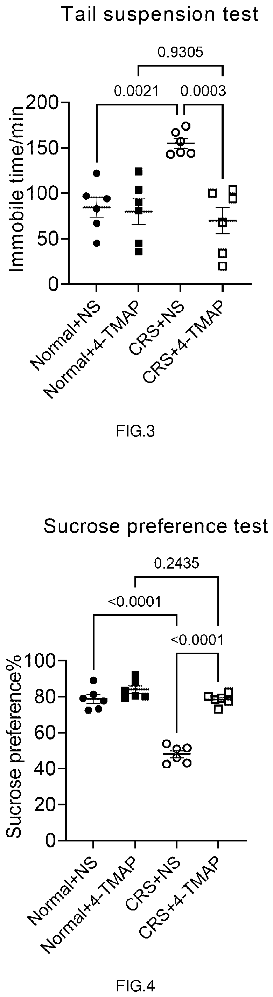

The results are shown in FIGs. 2 and 3. The immobility time of the mice in CRS + 4-TMAP group was significantly lower than that of the mice in CRS + normal saline group and was basically the same as the normal mouse group, indicating that 4-TMAP can significantly alleviate the depressive-like behavior of mice caused by CRS.

The sucrose preference test is used to evaluate the sucrose water preference of mice, which can reflect the degree of anhedonia of mice. In the entire animal experiment, a total of two sucrose preference tests were conducted. Before modeling, the sucrose preference coefficient of all mice was measured by the sucrose preference tests to ensure that the mice in the experiment were in the same state. After the test, the sucrose preference tests were formally carried out, including the adaptation training part and the test part. In the training, two bottles of 1% (w/v) sucrose solution were placed in each cage of mice in the first 24 hours, and one of the bottles was replaced with pure water in the next 24 hours. After the adaptation, fasting and water deprivation for 24 hours, and then the sucrose preference coefficient was measured. In the test, mice can only choose two bottles weighed in advance, one bottle is 1% (w/v) sucrose solution, and the other bottle is pure water. After fasting for 24 hours, take away the two bottles and weigh them, and record the total liquid consumption, sucrose water consumption and pure water consumption of the mice. The calculation method of sucrose preference coefficient is: sucrose preference coefficient (%) = sucrose water consumption/ (sucrose water consumption + pure water consumption) × 100%. The experimental results are shown in FIG. 4. The sucrose preference of the mice in CRS + 4-TMAP group was significantly higher than that of the mice in CRS + normal saline group (P<0.01) and was basically equivalent to that of the normal mouse group, indicating that 4-TMAP can significantly restore the anhedonia-like depressive behavior of mice caused by CRS.

### Example 3: Effect of 4-TMAP in a mouse model of chronic depression induced by corticosterone (Cort)

Twenty-four 6-8 week old SPF grade C57/B6j male mice (purchased from GemPharmatech) were randomly divided into four groups: one group was a normal mouse + normal saline group (6 mice), one group was a normal mouse + 4-TMAP group (6 mice), one group was a Cort mouse + normal saline group (6 mice), and one group was a Cort mouse + 4-TMAP group (6 mice). After adapting to the environment, from day 0, the drinking water of the Cort mouse group (MCE, HY-B1618) was a corticosterone solution with a final concentration of 25µg/mL (pH 7.0-7.4), and the normal mouse group received ordinary drinking water, which was changed every 2 days, and the model was established for 21 days. After the model was established, the depression modeling was confirmed to be successful by forced swimming, tail suspension, and sucrose preference tests (significant differences from normal mice).

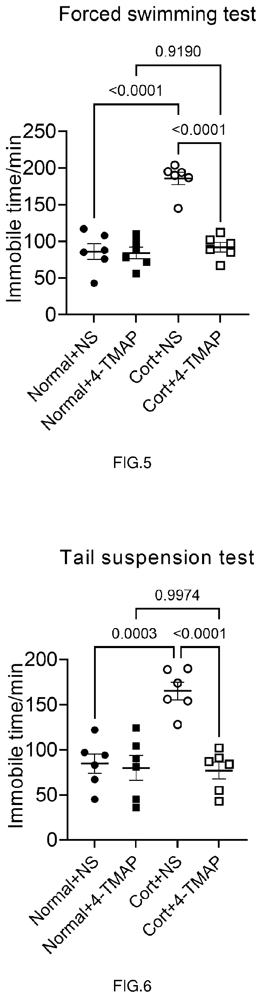

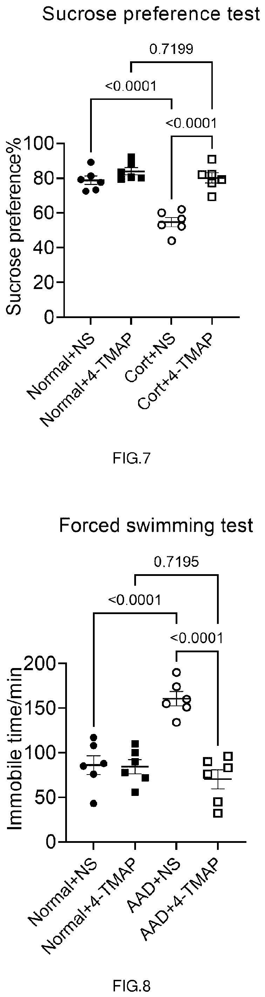

The mice in the 4-TMAP group were given 200 µL 4-TMAP (0.1 mg/kg) by intravenous injection, and the mice in the normal saline group were given 200 µL saline. 2 hours after administration, the mice were tested in the forced swimming and tail suspension tests, and the immobility time of the mice in each group in the forced swimming and tail suspension tests was compared. The results are shown in FIGs. 5 and 6. The immobility time of the Cort mice + 4-TMAP group was significantly lower than that of the Cort mice + normal saline group, which was basically the same as that of the normal mice group, indicating that 4-TMAP can significantly alleviate the depressive-like behavior of mice caused by corticosterone. The result of the sucrose preference test is shown in FIG. 7. The sucrose preference of the Cort mice + 4-TMAP group was significantly higher than that of the Cort mice + normal saline group (P<0.01), which was basically the same as that of the normal mice group, indicating that 4-TMAP can significantly restore the anhedonia-like depressive behavior of mice caused by Cort.

### Example 4: Effect of 4-TMAP in a mouse model of chronic depression induced by amino acid diet (AAD)

Twenty-four 6-8 week old SPF grade C57/B6j male mice (purchased from GemPharmatech) were randomly divided into four groups: one group was a normal mouse + normal saline group (6 mice), one group was a normal mouse + 4-TMAP group (6 mice), one group was a AAD mouse + normal saline group (6 mice), and one group was a AAD mouse + 4-TMAP group (6 mice). After adapting to the environment, from day 0, the normal mice group was fed with ordinary mouse feed, and the AAD mice group was fed with amino acid food (Medicience, MD12062, amino acid diet) for 4 consecutive weeks. Forced swimming, tail suspension, and sucrose preference tests were used to confirm that the depression model was successfully established (there were significant differences compared with normal mice).

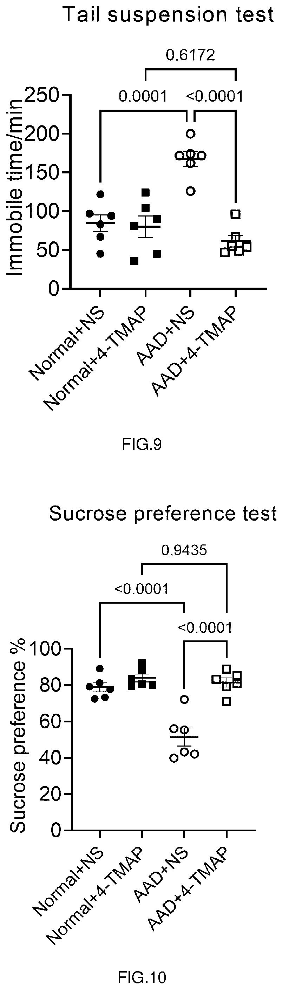

The mice in the 4-TMAP group were given 200 µL 4-TMAP (0.1 mg/kg) by intravenous injection, and the mice in the normal saline group were given 200 µL saline. 2 hours after administration, the mice were tested in the forced swimming and tail suspension tests, and the immobility time of the mice in each group in the forced swimming and tail suspension tests was compared. The results are shown in FIGs. 8 and 9. The immobility time of the AAD mice + 4-TMAP group was significantly lower than that of the AAD mice + normal saline group, which was basically the same as that of the normal mice group, indicating that 4-TMAP can significantly alleviate the depressive-like behavior of mice caused by corticosterone. The result of the sucrose preference test is shown in FIG. 10. The sucrose preference of the AAD mice + 4-TMAP group was significantly higher than that of the AAD mice + normal saline group (P<0.01), which was basically the same as that of the normal mice group, indicating that 4-TMAP can significantly restore the anhedonia-like depressive behavior of mice caused by AAD.

### Example 5: Minimum dose of 4-TMAP alleviates depressive-like behavior in mice

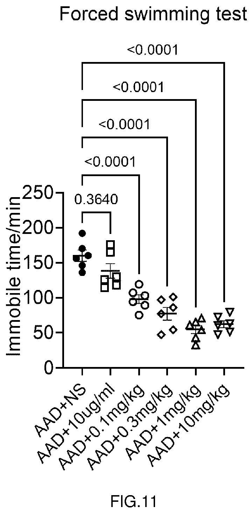

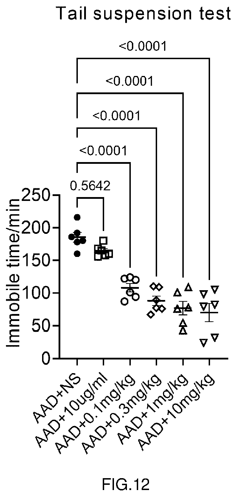

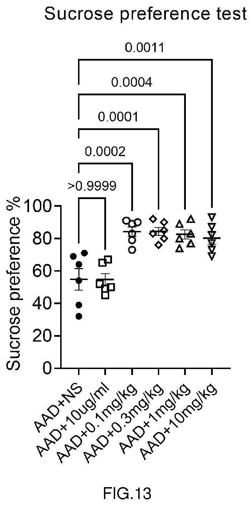

In a mouse model of depression induced by AAD, 4-TMAP was intravenously injected at different doses of 10 µg/kg, 0.1 mg/kg, 0.3 mg/kg, 1 mg/kg, and 10 mg/kg. 2 hours after administration, the minimum dose of 4-TMAP for antidepressant effect was confirmed by forced swimming, tail suspension test, and sucrose preference test. The experimental results are shown in FIGs. 11, 12, and 13. 4-TMAP can significantly reduce the immobility time of mice at concentrations of 0.1 mg/kg, 0.3 mg/kg, 1 mg/kg, and 10 mg/kg, and exerts an antidepressant effect at 0.1 mg/kg, indicating that the minimum therapeutic dose of 4-TMAP is 0.1 mg/kg. At the same time, it still has a significant antidepressant effect at concentrations of 0.3 mg/kg, 1 mg/kg, and 10 mg/kg.

### Example 6: Duration of a single dose of 4-TMAP alleviates depressive-like behavior in mice

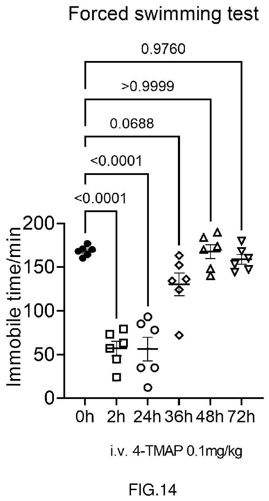

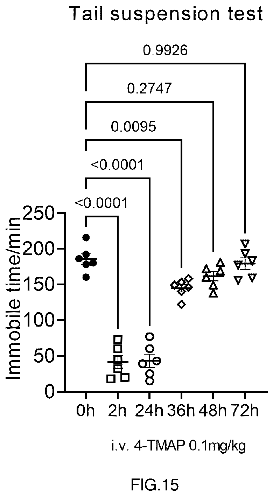

In a mouse model of depression induced by AAD, mice were intravenously injected with 4-TMAP (0.1 mg/kg). The duration of the antidepressant effect of 4-TMAP after a single administration was monitored by forced swimming and tail suspension tests at 2h, 24h, 36h, 48h, and 72h after administration. The experimental results are shown in FIGs. 14 and 15: 4-TMAP can significantly reduce the immobility time of mice at 2h and 24h after administration and tends to reduce the immobility time of mice at 36h but loses its effect at 48h and 72h. T his suggests that the duration of the antidepressant effect of 4-TMAP in mice after a single administration is 24-36h.

### Example 7: Comparison of the antidepressant effects of 4-TMAP and Brexpiprazole

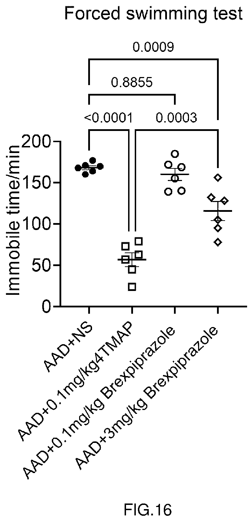

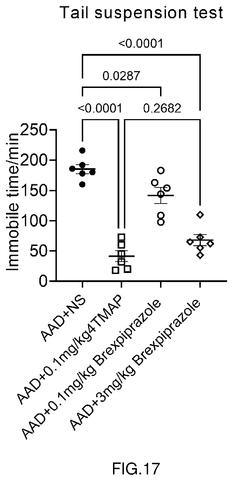

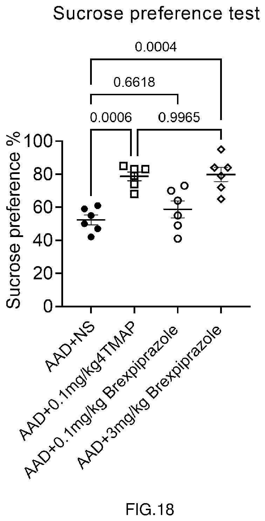

In a mouse model of depression induced by AAD, mice were intravenously injected with 4-TMAP (0.1mg/kg) and Brexpiprazole (0.1mg/kg, 3mg/kg). 2 hours after administration, forced swimming, tail suspension, and sucrose preference tests were used to compare the antidepressant effects of 4-TMAP and Brexpiprazole. The experimental results are shown in FIGs. 16, 17 and 18: Brexpiprazole cannot change the depressive-like behavior of mice at a dose of 0.1 mg/kg. However, when the dose of Brexpiprazole reached 3 mg/kg, it had the function of significantly reducing the immobility time of mice, similar to that of 4-TMAP at a dose of 0.1 mg/kg, indicating that 4-TMAP has a lower effective concentration than Brexpiprazole.

The basic principles and main features of the present invention and the advantages of the present invention have been shown and described above. It should be understood by those skilled in the industry that the present invention is not limited to the above embodiments. The above embodiments and descriptions are only for explaining the principles of the present invention. Without departing from the spirit and scope of the present invention, the present invention may have various changes and improvements, which fall within the scope of the present invention to be protected. The scope of protection of the present invention is defined by the appended claims and their equivalents.

## Claims

1. Use of 4-(trimethylammonio) pentanoate (4-TMAP) or its pharmaceutically acceptable salts in the preparation of drugs for treating or alleviating depression.

2. Use of 4-(trimethylammonio) pentanoate (4-TMAP) or its pharmaceutically acceptable salts in the preparation of drugs for treating or alleviating depression in mammals.

3. The use according to claim 2, **characterized in that** the mammals include primates or rodents.

4. The use according to any one of claims 2-3, **characterized in that** the mammals include humans, monkeys, or mice.

5. A pharmaceutical composition, **characterized by** 4-(trimethylammonio) pentanoate (4-TMAP) or its pharmaceutically acceptable salts as an active ingredient.

6. The pharmaceutical composition according to claim 5, **characterized in that** the pharmaceutical composition contains a therapeutically effective amount of 4-(trimethylammonio)pentanoate (4-TMAP) or its pharmaceutically acceptable salts.

7. The pharmaceutical composition according to claim 6, **characterized in that** the therapeutically effective amount is ≥0.1 mg of raw material drug per kg of mammal per dose.

8. The pharmaceutical composition according to any one of claims 5-7, **characterized in that** the pharmaceutical composition further comprises a pharmaceutically acceptable excipients, carriers, adjuvants, or media.

9. The pharmaceutical composition according to any one of claims 5-8, **characterized in that** the pharmaceutical composition is selected from injectable formulations or oral formulations.

10. Use of the pharmaceutical composition according to any one of claims 5-9 in the preparation of drugs for treating or alleviating depression.
